# EUROPÄISCHE PATENTANMELDUNG

(11) **EP 1 405 599 A1**
(43) Veröffentlichungstag der Anmeldung: **07.04.2004**
(21) Anmeldenummer: 02022415.0
(22) Anmeldetag: 04.10.2002
(51) Int. Cl.: A61B 10/00

(54) **Vorrichtung mit Ventilationsöffnungen zur Entnahme von Schleimhaut, Speichel, Sekret aus der Mundhöhle, aus anderen Körperöffnungen sowie Sekret aus Operationskörperöffnungen**

(71) Anmelder: bj-diagnostic GmbH, 35394 Giessen (DE)
(72) Erfinder: Bernhardt, Frank, Dr., 0000000 (DE)
(74) Vertreter: Goroll, Peter

(57) **Zusammenfassung**

Vorliegende Erfindung ist mit Ventilationsöffnungen zur Entnahme von Schleimhaut, Speichel, Sekret aus der Mundhöhle, aus anderen Körperöffnungen versehen und mit einem sterilen Tupfer (Wattestäbchen) (Fig. 1) (1) und der Verschlusskappe (Fig. 1) (2) zentrisch, aber auch exzentrisch verbunden. Diese Verschlusskappe (Fig. 1) (2) ist mit Durchbrüchen unterschiedlicher Art wie z. B. runden, ovalen, dreieckigen, viereckigen, fünfeckigen, sechseckigen, achteckigen oder noch mehreckigen oder mit längs-, kreuzoder halbrund angeordneten Durchbrüchen (Fig. 1) (3) versehen, so dass diese Vorrichtung mit den Ventilationsöffnungen das Sekret über einen langen Zeitraum hin durch die Ventilation haltbar macht.

## Beschreibung

Mit der Entzifferung von kompletten Genomen von verschiedenen Organismen (Human, Tier, Pflanze etc.) und der fortschreitenden Entschlüsselung der jeweiligen Genome (z. B. Zuordnung von Nukleotidsequenzen zu Genen o. ä.), wird das sich daraus ergebende Wissen über die Bedeutung z. B. in der medizinischen Diagnostik eingesetzt. Zur Zeit gehört die genetische Diagnostik noch nicht zur täglichen Routine in der medizinischen Diagnostik. DNA-Diagnostik, also die direkte Untersuchung der Basensequenz auf einem bestimmten Genort (locus), geschieht zur Zeit fast ausschließlich anhand von Blutproben, die dem Patienten zu diesem Zweck entnommen werden. Neben der direkten Basensequenz kann Information zu einem Genort dabei auch durch ein DNA-Fragment aus diesem Genort erlangt werden. Solche Fragmente können z. B. durch NAT Nukleinsäureamplifikationstechniken spezifisch erzeugt werden. Nukleinsäure befindet sich in allen Geweben oder Sekreten von Menschen, Tier, Pflanze oder Mikroorganismen. Daher kann neben Blut jedes andere Gewebe oder Sekret sowie Ausscheidungen (z. B. Speichel, ausgeatmete Luft, Schweiß, Urin, Kot etc.) von Organismen Nukleinsäure enthalten.

Zum Stand der Technik gehört heute, dass zur Gewinnung von Nukleinsäureproben zu diagnostischen Zwecken routinemäßig Blut Verwendung findet. Bei Neugeborenen wird zur Zeit vereinzelt bereits ein Abstrich aus der Mundhöhle mit einem Wattestäbchen gewonnen. Bei der Entnahme von Mundschleimhaut durch einen mechanischen Vorgang mit einem Wattestäbchen auf der Schleimhaut in der Mundhöhle (hin- und herreiben des Wattestäbchens auf der Schleimhaut) lösen sich Epithelzellen ab. Diese Zellen enthalten Zellkerne und somit Nukleinsäure. Dieser Vorgang ist nicht schmerzhaft und bietet durch die Verwendung steriler Wattestäbchen weit weniger Infektionsgefahr für den Patienten als eine Blut- oder Gewebeentnahme. Genomische Nukleinsäure eines Patienten aus Blut- oder Speichelproben (Mundschleimhautabrieb) gewonnen, ist vollständig identisch, d. h. es spielt keine Rolle aus welchem Probenmaterial genomische DNA gewonnen wird.

Die Nukleinsäurediagnostik wird seit einigen Jahren im Bereich der humanen Identifikation (Abstammungsuntersuchung, z. B. Vaterschaftsgutachten, Vaterschaftstest) in der Regel durch einen Mundschleimhautabrieb ausgeführt. Dabei ergeben sich bei der Verwendung üblicher Wattestäbchen verschiedene Probleme:

Die Wattestäbchen werden nach der Entnahme der Mundschleimhautprobe in der Regel zum Transport in ein Plastikröhrchen verpackt. Das mit Flüssigkeit (Speichel) vollgesaugte Wattestäbchen kann daher im Plastikröhrchen nicht trocknen. Dies entspricht dem Stand der Technik. Bei Raumtemperatur können sich in einem solchen Mikroklima Mikroorganismen (z. B. Bakterien oder Pilze), die auf der Mundschleimhaut vorkommen, sehr schnell vermehren. Dadurch werden die vorhandenen Zellen der Mundschleimhaut zerstört. Der Nukleinsäurenachweis im Labor liefert dann nur noch sehr schlechte Messwerte, die in der Regel keine diagnostische Aussage mehr zulassen.

Zu dem Stand der Technik gehört, dass medizinische Proben, wie Gewebe oder Abstrichmaterial, in der pathologischen Praxis immer archiviert werden. Neben rechtlichen spielen dabei auch medizinische Gründe eine Rolle. Ein einmal festgestelltes Untersuchungsergebnis kann daher jederzeit durch eine Neuuntersuchung reproduziert und bestätigt oder ergänzt werden. Archivierte Abstrichproben können aber auch erneut untersucht werden, wenn sich neue wissenschaftliche Erkenntnisse ergeben, z. B. eine neue Technik, die mehr Information mit medizinischer oder forensischer Bedeutung liefert. Gerade am Beispiel von DNA-Datenbanken , wie sie bei national und internationalen Polizeibehörden vorhanden sind, zeigt sich, dass archivierte Proben erneut untersucht werden müssen, wenn eine neue Untersuchungstechnik/Technologie zur Verfügung steht. Dadurch ergeben sich weitere Erkenntnisse zur Identität einer Person. Wird eine alte Technik durch eine neue ersetzt, müssen auch die archivierten Proben mit der neuen Technik analysiert werden. Dazu wird heute in der Regel archivierte, isolierte DNA benötigt. Die von der Zelle getrennte, isolierte Nukleinsäure ist aber instabil und nur über mehrere Jahre haltbar, wenn sie bei mindestens -80 Celsius gelagert wird. Dies ist ein wenig praktikabeles und sehr kostenintensives Verfahren bei dem erhebliche Anschaffungskosten der Kühlsysteme, Stromkosten, Raumkosten und Wartung anfallen. Diese Kosten können mit der Lagerung von Zellen nach dem oben beschriebenen Abstrichsystem mit der neuartigen Vorrichtung mit Ventilationsöffnungen oder dem gleichen zu erreichenden Effekt mit dem Dehydrierungsverfahren erheblich gesenkt werden.

Nachfolgende Innovationen werden wie folgt beschrieben:

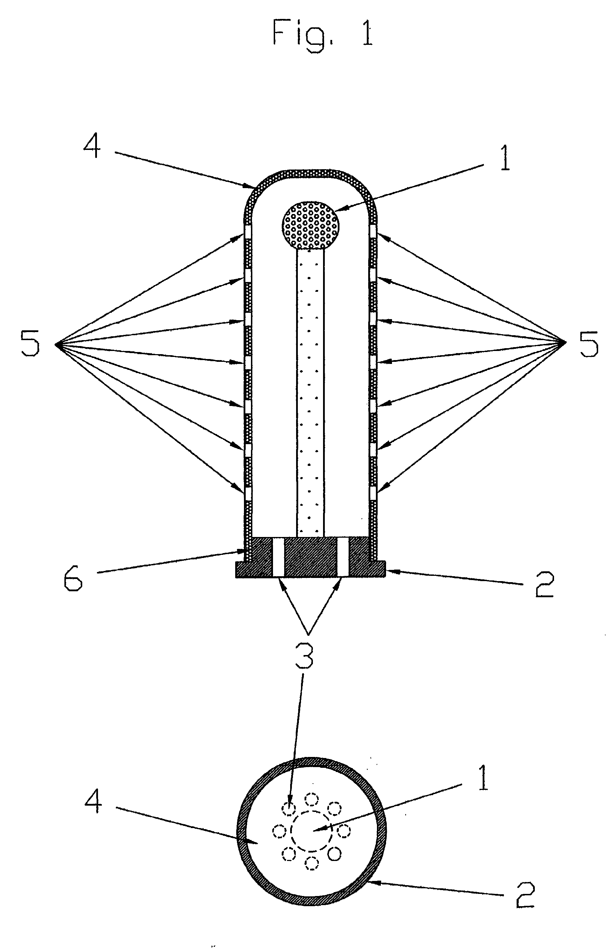

Der sterile Tupfer (Wattestäbchen) (Fig. 1) (1) ist mit der Verschlusskappe (Fig. 1) (2) zentrisch verbunden, er kann auch exzentrisch angebracht werden.

Diese Verschlusskappe (Fig. 1) (2) kann mit Durchbrüchen unterschiedlicher Art wie z. B. runden, ovalen, dreieckigen, viereckigen, sechseckigen, achteckigen oder noch mehreckigen oder mit längs- oder mit kreuz- oder halbrund angeordneten Durchbrüchen (Fig. 1)(3) versehen sein.

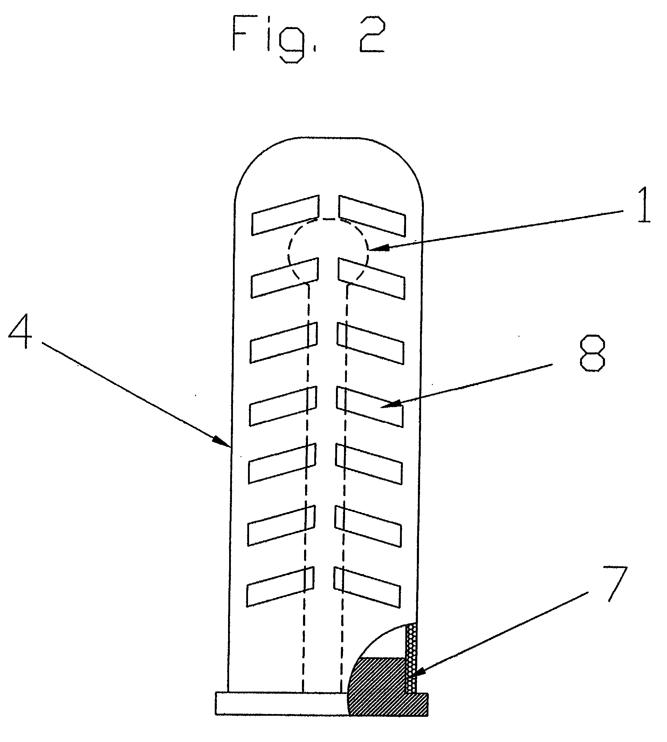

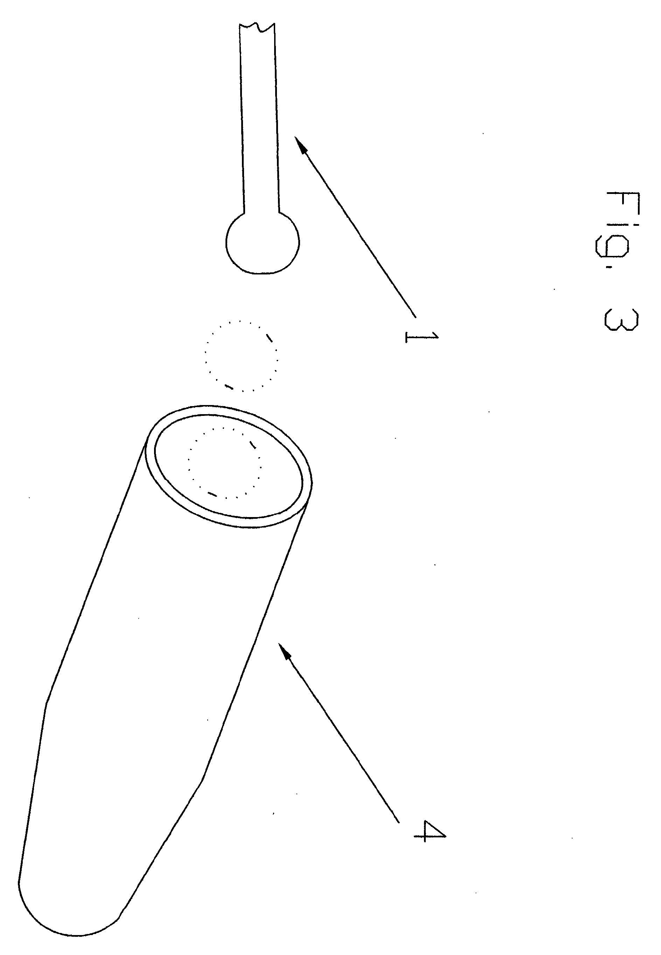

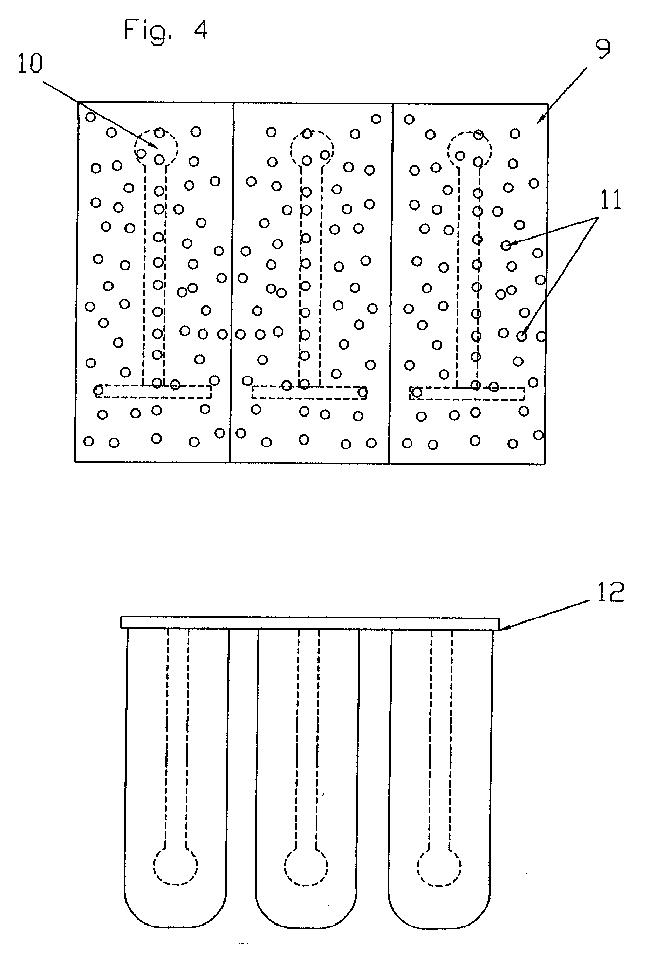

Der Verschlusskappe ist das dazugehörige Verschlussröhrchen (Fig. 1) (4) integriert. Dieses Verschlussröhrchen (Fig. 1) (4) ist mit unterschiedlichen Durchbrüchen versehen. Die Durchbrüche sind an der Seitenwandung angebracht (Fig. 1) (5). Sie bewirken die Ventilierung des sterilen Tupfers (Fig. 1) (1) über das Verschlussröhrchen (Fig. 1) (4). Sowohl das Verschlussröhrchen (Fig. 1) (4) als auch die Verschlusskappe (Fig. 1) (2) können aus Kunststoff, Plexiglas, Polyethylen gefertigt sein. An der Schnittstelle (Fig. 1) (6) wird das Verschlussröhrchen (Fig. 1) (4) auf die Verschlusskappe (Fig. 1) (2) aufgepresst damit der damit verbundene Tupfer fest verbunden mit der Verschlusskappe (Fig. 1) (2) fest in dem Röhrchen sitzt ohne die Wandungen des Röhrchens zu berühren. Die Schnittstelle (Fig. 1) (6) der Verbindungen des Röhrchens (Fig. 1) (4) und Verschlusskappe (Fig. 1) (2) kann auch mittels eines Gewindes (Fig. 2) (7) erfolgen.

(Fig. 2) (8) zeigt einen der möglichen Durchbrüche, die in ihrer Vielzahl zuvor beschrieben wurden.

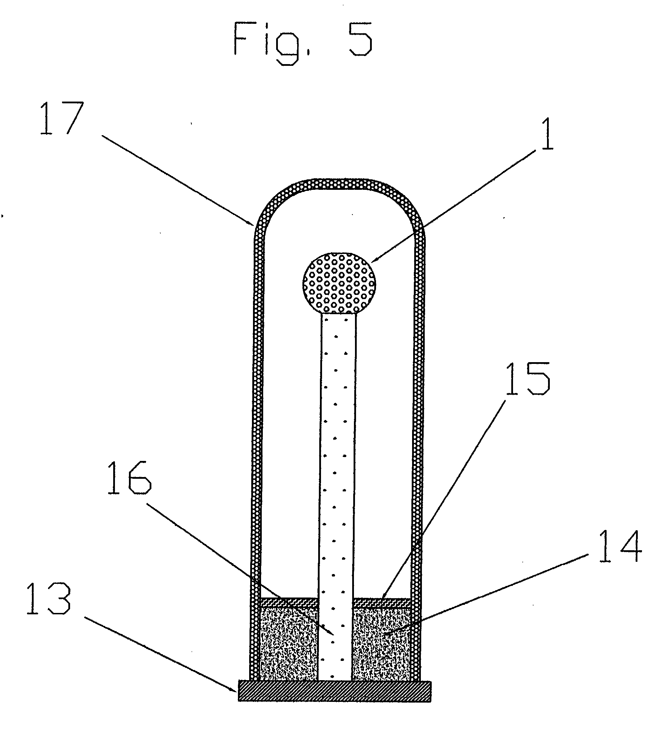

Der Abstrichkörper (Fig. 2) (Fig. 3) (1) nimmt den Speichel oder die ähnlichen Körperflüssigkeiten auf und wird in das Röhrchen (Fig. 3) (4) entnommen.

Nach einem Abstrich kann die Abstrichvorrichtung (Fig. 1) (1) in eine Hülle (Fig. 4) (9) gelegt oder gesteckt werden, um einen kontaminationsreichen Transport oder eine kontaminationsfreie Lagerung zu ermöglichen. Gleiches kann auch durch eine mit der Abstrichvorrichtung fest verbundene Hülle (Fig. 1) (4) erreicht werden. Diese Hülle (Fig. 1) (4) kann nach der Entnahme (Mundschleimhaut, Speichel) über die Abstrichkörper (Fig. 1 ) (1) geschoben werden.

Die Verpackung (Fig. 1) (4) (Fig. 4) (9) zum Transport oder zur Lagerung erfolgt mit nicht luftdicht verschlossenen Hüllen (Kunststoff, Papier etc.). Die Art der Verpackung ist daher dann dadurch gekennzeichnet, dass sie die feuchte, entnommene Probe während des Transportes oder der Lagerung belüftet (Fig. 1) (3 + 6) (Fig. 4) (11) und somit trocknet. Es gibt zwei Systeme:
a) belüftet
b) mit Trockenmittel (geschlossen)

Die nach der Entnahme getrocknete Probe ist an einem trockenen, dunkeln Ort aufbewahrt über Jahre oder Jahrzehnte haltbar. Liegt die Lagertemperatur weit über 20 Grad Celsius, bei hoher Luftfeuchtigkeit, kann der Abstrichkörper (Fig. 1) (1) der Probe entsprechend diagnostischen Anforderung ganz oder teilweise mit einem Konservierungsstoff (z. b. Askorbinsäure (Fig. 4) (10)) vor Entnahme der Probe aus der Mundhöhle behandelt werden. Das entnommene Material (Zellen, Speichel) kann auf diese Weise fixiert und über lange Zeit bei Raumtemperatur haltbar gemacht werden.

Die eingetrockneten Zellen stellen einen fixierten Zustand dar, hingegen bleibt bereits isolierte DNA nur bei -80 Grad Celsius lange Zeit stabil. Da die Abstrichträger sehr klein sind, ist die platzsparende Lagerung verglichen mit einer Lagerung bei -80 Grad Celsius extrem kostensparend.

Sollten mehrere Abstrichvorrichtungen (Fig. 4) (12) einer bestimmten Gruppe von Testpersonen (z. B. einer Familie) zugeordnet werden, können z. B. mehrere zylindrische Hüllen miteinander festverbunden werden.

Mit der Dehydrierungsvorrichtung wird der selbe Effekt erreicht, wie mit der Ventilationsvorrichtung (Fig. 1). Die Dehydrierungsvorrichtung ergibt sich aus Figur 5 wie folgt:

Der Verschlusskappe (Fig. 5) (13) ist die Dehydrierungskammer (Fig. 5) (14) integriert. Die Dehydrierungskammer ist mit einer Kappe (Fig. 5) (15) versehen, die die Dehydrierungskammer (Fig. 5) (14) verschließt.

In der Dehydrierungskammer (Fig. 5) (14) befindet sich wasseraufnehmendes Granulat (Fig. 5) (16), welches aus einer hygroskopischen Substanz (z. B. Silicagel) sowie ähnlichen Stoffen bestehen kann.

In der Dehydrierungskammer (Fig. 5) (14) ist die Abstrichvorrichtung/Wattestäbchen (Fig. 5) (1) zum Abstrich befestigt.

Nach der Speichel- respektive Zellentnahme wird der Verschlusskopf (Fig. 5) (13) mit dem integrierten Dehydrierungsgranulat durch die Hülle (Fig. 5) (17) luftdicht verschlossen.

Die vorhandene Feuchtigkeit an der Abstrichvorrichtung (Fig. 5) (1) wird durch die Dehydrierungsvorrichtung (Fig. 5) (14, 15, 16) aufgenommen, so dass über einen langen Zeitraum der Abstrichkörper ohne Lagerung bei -80 Grad Celsius aufgehoben und archiviert werden kann.

## Patentansprüche

1. Vorrichtung mit Ventilationsöffnungen zur Entnahme von Schleimhaut, Speichel, Sekret aus der Mundhöhle, aus anderen Körperöffnungen sowie Sekret aus Operationskörperöffnungen **dadurch gekennzeichnet, dass** der sterile Tupfer (Wattestäbchen) (Fig. 1) (1) mit der Verschlusskappe (Fig. 1) (2) zentrisch aber auch exzentrisch verbunden ist sowie diese Verschlusskappe (Fig. 1) (2) mit Durchbrüchen unterschiedlicher Art wie z. B. runden, ovalen, dreieckigen, viereckigen, fünfeckigen, sechseckigen, achteckigen oder noch mehreckigen oder mit längs- kreuz- oder halbrund angeordneten Durchbrüchen (Fig. 1) (3) versehen ist und das dazugehörige Verschlussröhrchen (Fig. 1) (4) integriert und mit unterschiedlichen Durchbrüchen versehen ist.

2. Vorrichtung mit Ventilationsöffnungen zur Entnahme von Schleimhaut, Speichel, Sekret aus der Mundhöhle, aus anderen Körperöffnungen sowie Sekret aus Operationskörperöffnungen nach Anspruch 1, **dadurch gekennzeichnet, dass** die Durchbrüche an der Seitenwandung angebracht (Fig. 1) (5) sind.

3. Vorrichtung mit Ventilationsöffnungen zur Entnahme von Schleimhaut, Speichel, Sekret aus der Mundhöhle, aus anderen Körperöffnungen sowie Sekret aus Operationskörperöffnungen nach Ansprüchen 1. und 2. **dadurch gekennzeichnet, dass** sowohl das Verschlussröhrchen (Fig. 1) (4) als auch die Verschlusskappe (Fig. 1) (2) aus Kunststoff, Plexiglas, Polyethylen gefertigt sind.

4. Vorrichtung mit Ventilationsöffnungen zur Entnahme von Schleimhaut, Speichel, Sekret aus der Mundhöhle, aus anderen Körperöffnungen sowie Sekret aus Operationskörperöffnungen nach Ansprüchen 1. bis 3. **dadurch gekennzeichnet, dass** an der Schnittstelle (Fig. 1) (6) das Verschlussröhrchen (Fig. 1) (4) auf die Verschlusskappe (Fig. 1) (2) aufgepresst oder aber auch mittels eines Gewindes (Fig. 2) (7) erfolgt.

5. Vorrichtung mit Ventilationsöffnungen zur Entnahme von Schleimhaut, Speichel, Sekret aus der Mundhöhle, aus anderen Körperöffnungen sowie Sekret aus Operationskörperöffnungen nach Ansprüchen 1. bis 4. **dadurch gekennzeichnet, dass** die Durchbrüche (Fig. 2) (8) rund, oval, dreieckig, viereckig, fünfeckig, sechseckig, achteckig und mehreckig oder mit längs- kreuzoder halbrund angeordneten Durchbrüchen versehen ist.

6. Vorrichtung mit Ventilationsöffnungen zur Entnahme von Schleimhaut, Speichel, Sekret aus der Mundhöhle, aus anderen Körperöffnungen sowie Sekret aus Operationskörperöffnungen nach Ansprüchen 1. bis 5. **dadurch gekennzeichnet, dass** der Abstrichkörper (Fig. 2 und 3) (1) den Speichel oder ähnliche Körperflüssigkeit aufnimmt und in das Röhrchen (Fig. 3) (4) entnommen wird.

7. Vorrichtung mit Ventilationsöffnungen zur Entnahme von Schleimhaut, Speichel, Sekret aus der Mundhöhle, aus anderen Körperöffnungen sowie Sekret aus Operationskörperöffnungen nach Ansprüchen 1. bis 6. **dadurch gekennzeichnet, dass** die Abstrichvorrichtung (Fig. 1) (1) in eine Hülle (Fig. 4) (9) gelegt oder gesteckt wird.

8. Vorrichtung mit Ventilationsöffnungen zur Entnahme von Schleimhaut, Speichel, Sekret aus der Mundhöhle, aus anderen Körperöffnungen sowie Sekret aus Operationskörperöffnungen nach Ansprüchen 1. bis 7. **dadurch gekennzeichnet, dass** die Verpackung (Fig. 4) (9) zum Transport oder zur Lagerung mit nicht luftdicht verschlossenen Hüllen (Fig. 1) (3), (Fig. 4) (11) versehen ist.

9. Vorrichtung mit Ventilationsöffnungen zur Entnahme von Schleimhaut, Speichel, Sekret aus der Mundhöhle, aus anderen Körperöffnungen sowie Sekret aus Operationskörperöffnungen nach Ansprüchen 1. bis 8. **dadurch gekennzeichnet, dass** der Abstrichkörper (Fig. 1) (1) ganz oder teilweise mit einem Konservierungsstoff (Fig. 4) (10) versehen wird.

10. Vorrichtung mit Ventilationsöffnungen zur Entnahme von Schleimhaut, Speichel, Sekret aus der Mundhöhle, aus anderen Körperöffnungen sowie Sekret aus Operationskörperöffnungen nach Ansprüchen 1. bis 9. **dadurch gekennzeichnet, dass** mehrere Abstrichvorrichtungen (Fig. 4) (12) fest miteinander verbunden sind.

11. Vorrichtung mit Ventilationsöffnungen zur Entnahme von Schleimhaut, Speichel, Sekret aus der Mundhöhle, aus anderen Körperöffnungen sowie Sekret aus Operationskörperöffnungen nach Ansprüchen 1, bis 10. **dadurch gekennzeichnet, dass** die Verschlusskappe (Fig. 5) (13) mit der Dehydrierungskammer (Fig. 5) (14) verbunden und diese mit einer Kappe (Fig. 5) (15) die Dehydrierungskammer (Fig. 5) (14) verschließt.

12. Vorrichtung mit Ventilationsöffnungen zur Entnahme von Schleimhaut, Speichel, Sekret aus der Mundhöhle, aus anderen Körperöffnungen sowie Sekret aus Operationskörperöffnungen nach Ansprüchen 1. und 11. **dadurch gekennzeichnet, dass** sich wasseraufnehmendes Granulat (Fig. 5) (16) in der Dehydrierungskammer (Fig. 5) (14) befindet.

13. Vorrichtung mit Ventilationsöffnungen zur Entnahme von Schleimhaut, Speichel, Sekret aus der Mundhöhle, aus anderen Körperöffnungen sowie Sekret aus Operationskörperöffnungen nach Ansprüchen 1., 11. und 12. **dadurch gekennzeichnet, dass** mit der Dehydrierungskammer (Fig. 5) (14) die Abstrichvorrichtung (Fig. 5) (1) befestigt ist sowie der Verschlusskopf (Fig. 5) (13) mit dem integrierten Dehydrierungsgranulat durch die Hülle (Fig. 5) (17) luftdicht verschlossen wird.
